# EUROPEAN PATENT SPECIFICATION

(11) **EP 4 574 030 B1**
(45) Date of publication and mention of the grant of the patent: **26.08.2026**
(21) Application number: 24221353.6
(22) Date of filing: 19.12.2024
(51) Int. Cl.: A61B 5/053, A61B 5/08, A61B 5/085, A61B 18/14, A61B 5/06

(54) **END-OF-EXPIRIUM DETECTION USING IMPEDANCE MEASUREMENTS**
NACHWEIS DES ENDES EINES EXPERRIUMS MITTELS IMPEDANZMESSUNGEN
DÉTECTION DE FIN D'EXPIRATION À L'AIDE DE MESURES D'IMPÉDANCE

(30) Priority: 20.12.2023 US 202318390188
(43) Date of publication of application: 25.06.2025
(73) Proprietor: Biosense Webster (Israel) Ltd., 2066717 Yokneam (IL)
(72) Inventor: RAZ, Shaul, 2066717 Yokneam (IL)
(74) Representative: Carpmaels & Ransford LLP

(56) References cited:
- EP-A1- 4 014 857
- EP-B1- 1 999 639
- US-A1- 2006 074 300
- US-B2- 10 524 692

## Description

### FIELD OF THE DISCLOSURE

The present disclosure relates generally to detection of respiration effects, and specifically to detecting the times of ends of expirium.

### BACKGROUND OF THE DISCLOSURE

Techniques to compensate for respiratory effects were previously reported in patent literature. For example, U.S. Patent 10,524,692 describes a method including positioning body-electrodes in galvanic contact with a body of a patient and locating a probe in the body of the patient. The method further includes tracking positions of the probe during respiration of the patient and determining indications related to impedances between the body-electrodes during the respiration. The method also includes calculating a function relating the positions of the probe to the indications, and applying the function to identify end-expirium points of the respiration based on subsequent indications related to the impedances.

In US10524692B2, there is described a method that includes positioning body-electrodes in galvanic contact with a body of a patient and locating a probe in the body of the patient. The method further includes tracking positions of the probe during respiration of the patient and determining indications related to impedances between the body-electrodes during the respiration. The method also includes calculating a function relating the positions of the probe to the indications, and applying the function to

identify end-expirium points of the respiration based on subsequent indications related to the impedances.

In EP1999639B1, there is described a cardiographic apparatus having a plurality of electrodes that are configured for operative connection with a subject. A cardiograph is connected with the plurality of electrodes to acquire cardiographic data. A respiratory gate is configured to identify end expiration periods or other quiescent respiratory periods so as to generate a cardiographic dataset limited to the identified end-expiration periods from cardiographic data acquired by the cardiograph at least during the identified end expiration periods.

In US2006074300A1, there is described a medical diagnostic imaging system that includes a diagnostic imaging scanner that acquires imaging data of a medical imaging patient. A reconstruction processor reconstructs at least a portion of the acquired imaging data into an image representation. A pair of electrodes contact a thoracic region of the patient. An electrical meter measures electrical resistance R(t) or another time-varying electrical parameter across the electrode pair during the acquiring of imaging data. A respiration monitor extracts a time-varying respiration characteristic from the measured time-varying electrical parameter to indicate respiratory cycle phase.

The present disclosure will be more fully understood from the following detailed description of the examples thereof, taken together with the drawings, in which:

### SUMMARY

The invention is defined by the appended independent claims. Further embodiments are defined by the dependent claims.

### BRIEF DESCRIPTION OF THE DRAWINGS

Fig. 1 is a schematic, pictorial illustration of a catheter-based electroanatomical (EA) mapping and ablation system, in accordance with an example of the present disclosure;
Fig. 2 is a schematic, pictorial illustration of layout and circuitry of an electrical measurement apparatus used in detection of end-of-expirium, in accordance with an example of the present disclosure;
Fig. 3 is a graph of the total impedance R(t) of an actual patient undergoing cardiac ablation, in accordance with an example of the present disclosure; and
Fig. 4 is a flow chart that schematically illustrates a method and algorithm to electrically detect end-of-expirium, in accordance with an example of the present disclosure.

### DETAILED DESCRIPTION OF EXAMPLES

### OVERVIEW

A wide range of medical procedures involve the use of invasive probes, such as cardiac catheters to diagnose and/or treat a patient. The quality of the medical application may depend on detection of the end of the exhalation part of the respiratory cycle (referred to herein as "end-of-expirium"). Timing a medical procedure to occur at the end-of-expirium is desirable, due to, for example, relaxation in internal motion. For example, applying cardiac ablation during end-of-expirium may be safer and more accurate.

During the breathing cycle, a body's measured electrical impedance tends to be affected by the lungs alternately being filled with, and then emptied of, air, since air is an insulator. Examples of the present disclosure that are described herein provide a technique to follow a total body impedance indication calculated from conductivities measured using patch electrodes attached to the skin of the patient's trunk (e.g., at the back and thorax regions). The disclosed total body impedance indication is found to be well correlated to lungs emptied of air at the end-of-expirium. In some examples, the disclosed technique utilizes several patch electrodes attached to the skin of a patient to measure a combined conductivity matrix **σ** that describes the conductance of the patch electrodes and a patient body's conductance.

The patch electrodes used can be these of an electrical tracking system already in place (e.g., to track the position of a catheter inside the heart). In such a case, each patch acts as a transmitter of an AC signal in a unique frequency, while the other patches act as receivers. In this way, the system can receive multiple AC signals, and based on these signals, a processor can calculate the conductivity matrix,

An example of a method and technique to obtain a combined conductivity matrix **σ** is described in U.S Patents No. 8,456,182 and 10,524,692, both assigned to the assignee of the current application. The inventors noted that a parameter best describing the subject's torso impedance is the sum over all patches of patch-to-patch conductivities. This sum can be obtained by calculating the diagonal sum (e.g., matrix trace) of conductivity matrix **σ.**

To this end, a processor uses the value of the reciprocal of the trace of matrix **σ**, *R*(t)=1/Tr(**σ**), where t is time. Times at which the lungs are most emptied of air (i.e., the ends-of-expirium) during respective respiration cycles are manifested as minima of the time-dependent total impedance function R(t).

The processor can output the timings of end-of-expirium to a user as an algorithm (e.g., by tagging the minima, and outputting the tags, to a cardiac ablation algorithm). Alternatively, or additionally, the processor may output the timings of end-of-expirium by displaying curve R(t) on a display device.

The disclosed end-of-expirium detection technique was found accurate and robust in the service of cardiac ablation, as well as other electrical noise-inducing phenomena during invasive and noninvasive treatments that require end-of-expirium detection. In general, the disclosed technique may be applied as described below, with a standalone processor or with a processor of any medical system that requires the use of the technique (e.g., a medical imaging system that requires respiratory gating).

### SYSTEM DESCRIPTION

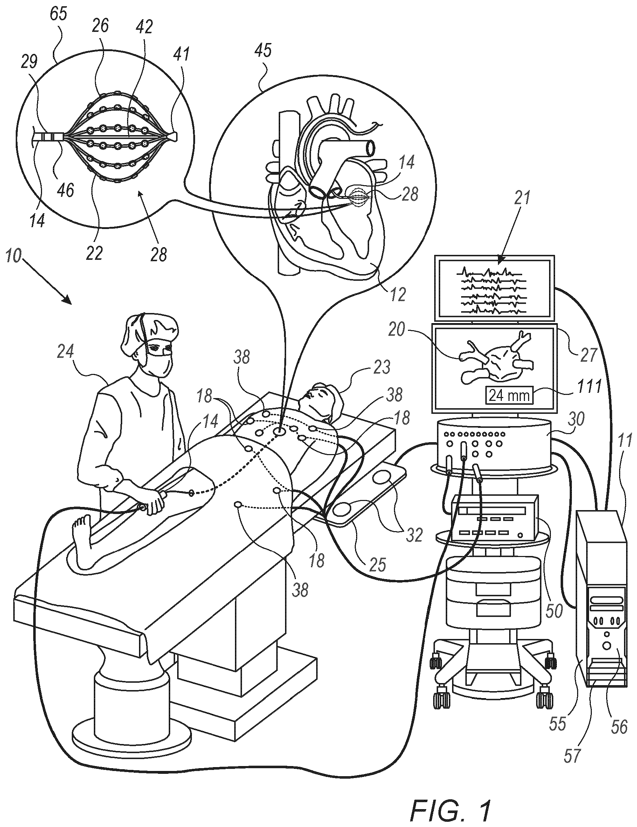

Fig. 1 is a schematic, pictorial illustration of a catheter-based electro-anatomical (EA) mapping and ablation system 10, in accordance with an example of the present disclosure.

System 10 includes multiple catheters which are percutaneously inserted by physician 24 through the patient's vascular system into a chamber or vascular structure of a heart 12 (seen in inset 45). Typically, a delivery sheath catheter is inserted into a cardiac chamber, such as the left or right atrium near a desired location in heart 12. Thereafter, a plurality of catheters is inserted into the delivery sheath catheter in order to arrive at the desired location. The plurality of catheters may include a catheter dedicated for pacing, a catheter for sensing intracardiac electrogram signals, a catheter dedicated for ablating and/or a catheter dedicated for both EA mapping and ablating. An example catheter 14, illustrated herein, is configured for sensing bipolar electrograms. Physician 24 brings a distal tip 28 (also called hereinafter distal end assembly 28) of catheter 14 into contact with the heart wall for sensing a target site in heart 12. For ablation, physician 24 similarly brings a distal end of an ablation catheter to a target site.

As seen in inset 65, catheter 14 is an exemplary catheter that includes a basket distal end 28, including one, and preferably multiple, electrodes 26 optionally distributed over a plurality of splines 22 at distal tip 28 and configured to sense IEGM signals. Catheter 14 may additionally include a position sensor 29 embedded in or near distal tip 28 on a shaft 46 of catheter 14, used to track the position and orientation of distal tip 28. Optionally, and preferably, position sensor 29 is a magnetic-based position sensor including three magnetic coils for sensing three-dimensional (3D) position and orientation. As seen, distal tip 28 further includes an expansion/collapse rod 42 of expandable assembly 28 that is mechanically connected to basket assembly 28 at a distal edge 41 of assembly 28.

Magnetic based position sensor 29 may be operated together with a location pad 25 that includes a plurality of magnetic coils 32 configured to generate magnetic fields in a predefined working volume. Real-time position of distal tip 28 of catheter 14 may be tracked based on magnetic fields generated with location pad 25 and sensed by magnetic based position sensor 29. Details of the magnetic based position sensing technology are described **in** U.S. Patent Nos. 5,391,199; 5,443,489; 5,558,091; 6,172,499; 6,239,724; 6,332,089; 6,484,118; 6,618,612; 6,690,963; 6,788,967; 6,892,091.

System 10 includes a plurality of electrode patches 38 positioned for skin contact on patient 23 to establish a location reference for location pad 25 as well as impedance-based tracking of electrodes 26. For impedance-based tracking, electrical current is directed toward electrodes 26 and sensed at electrode skin patches 38 so that the location of each electrode can be triangulated via electrode patches 38. Details of the impedance-based location tracking technology are described in US Patent Nos. 7,536,218; 7,756,576; 7,848,787; 7,869,865; 8,456,182; and 10,524,692.

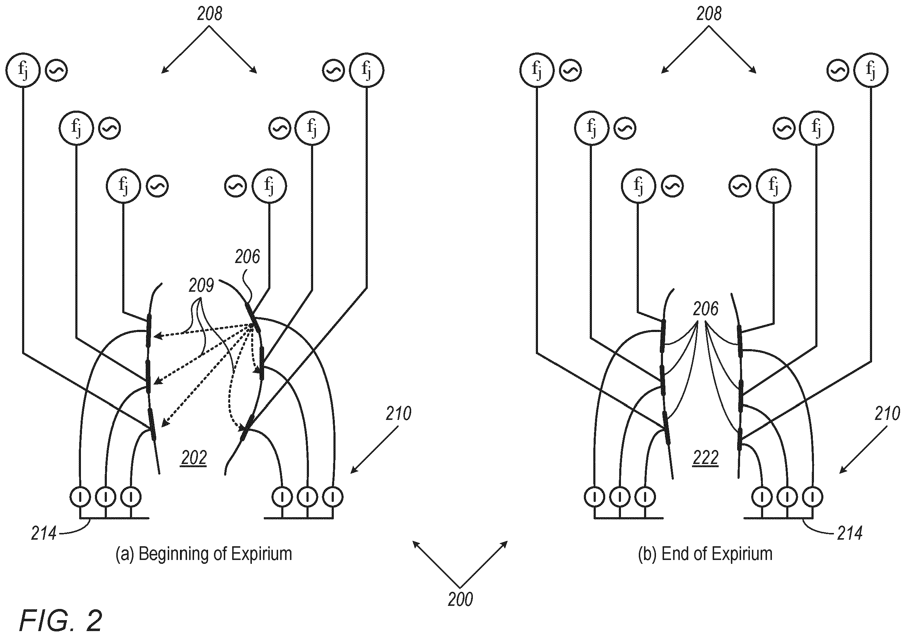

Electrode patches 38 can be used, in a stand-alone fashion with a processor running the disclosed algorithm, to electrically detect an end-of-expirium, as described in Figs. 2 to **4****.** This detection is used to improve the outcome of an ablation using a probe such as catheter 14, but the detection itself does not depend on the presence of a probe inside the patient's body. In a disclosed example, physician 24 may select/unselect and/or observe results of the disclosed end-of-expirium detection algorithm, e.g., via a graphical user interface (GUI) 111.

A recorder 11 displays, on display device 27, cardiac signals 21 (e.g., electrograms acquired at respectively tracked cardiac tissue positions) acquired with body surface ECG electrodes 18 and intracardiac electrograms acquired with electrodes 26 of catheter 14. Recorder 11 may include pacing capability to pace the heart rhythm, and/or may be electrically connected to a standalone pacer.

Workstation 55 includes memory 57, a processor 56 unit with memory or storage with appropriate operating software loaded therein, and user interface capability. Workstation 55 may provide multiple functions, optionally including (i) modeling endocardial anatomy in three-dimensions (3D) and rendering the model or EA map 20 for display on display device 27, (ii) displaying activation sequences (or other data) compiled from recorded cardiac signals 21 in representative visual indicia or imagery superimposed on the rendered EA map 20 on display device 27, (iii) displaying real-time location and orientation of multiple catheters within the heart chamber, and (iv) displaying sites of interest on display device 27, such as places where ablation energy has been applied. One commercial product embodying elements of system 10 is available as the CARTO^{™} 3 System, available from Biosense Webster, Inc., 31A Technology Drive, Irvine, CA 92618.

System 10 may include an ablation energy generator 50 that is adapted to conduct ablative energy to one or more electrodes at a distal tip of a catheter configured for ablation. Energy produced by ablation energy generator 50 may include, but is not limited to, radiofrequency (RF) energy or pulsed-field ablation (PFA) energy, including monopolar or bipolar high-voltage DC pulses, to be used to effect irreversible electroporation (IRE), or combinations thereof.

Patient interface unit (PIU) 30 is an interface configured to establish electrical communication between catheters, electrophysiological equipment, power supply and a workstation 55 to control system 10 operation and to receive EA signals from the catheter. Electrophysiological equipment of system 10 may include, for example, multiple catheters, location pad 25, body surface ECG electrodes 18, electrode patches 38, ablation energy generator 50, and recorder 11. Optionally, and preferably, PIU 30 additionally includes processing capability for implementing real-time computations of catheter locations and for performing ECG calculations.

In some examples, processor 56 typically comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

This configuration of system 10 is shown by way of example, in order to illustrate certain problems that are addressed by examples of the present disclosure and to demonstrate the application of these examples in enhancing the performance of such a system. Examples of the present disclosure, however, are by no means limited to this specific sort of example system, and the principles described herein may similarly be applied to other types of medical systems. For example, other multi-electrode catheter types may be used, such as the multi-arm OCTARAY^{™} catheter or a flat catheter.

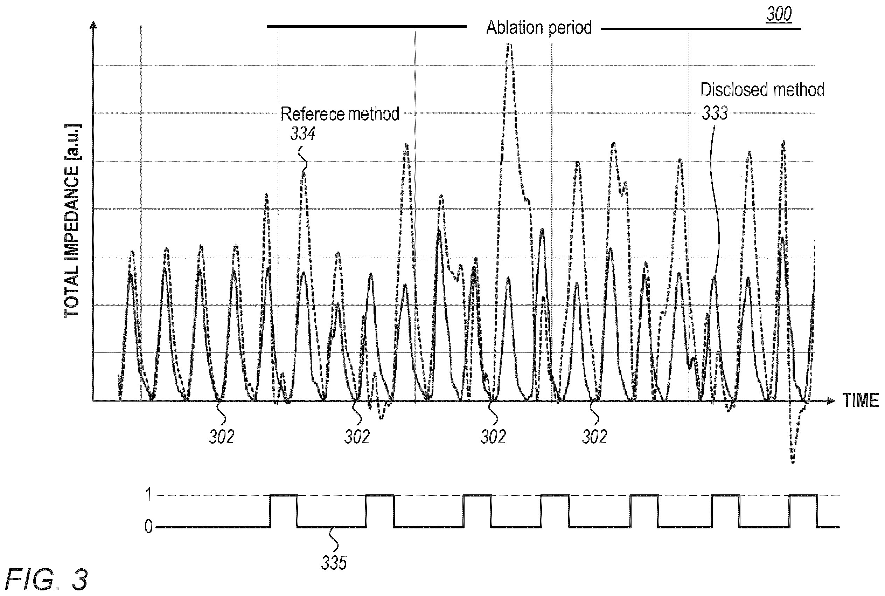

While the disclosed technique is described using Fig. 1, the technique can be described using Fig. 2 below with a standalone processor, without any relation to Fig. 1. As such, the disclosed technique may be applied as described in Figs. 2 and 3, with the standalone processors, or with a processor of any medical system that requires the use of the technique (e.g., a medical imaging system that requires respiratory gating).

### ELECTRICAL MEASUREMENT APPARATUS TO DETECT END-OF-EXPIRIUM

Fig. 2 is a schematic pictorial drawing of layout and circuitry of an electrical measurement apparatus 200 used to detect end-of-expirium, in accordance with an example of the present disclosure. Apparatus 200 is seen in two states: (a) beginning of expirium and (b) end-of-expirium. The difference between the two states is the amount of air inside the lungs, schematically illustrated by a first side view 202 of the torso as seen in the beginning of expirium and a second side view 222 of the torso as seen in the end-of-expirium. As further seen, there are six patch electrodes 206 in use by apparatus 200, of which three are attached to the skin at the back of the patient's trunk (202, 222) and three to the skin at the chest.

AC signal sources 208 apply an AC signal between each respective patch electrode 206 and a common ground 214. AC signal meters 210 measure resulting AC signals between each of the patch electrodes 206 and the common ground 214. AC signal generator sources 208 each transmit at a unique frequency fⱼ. The body's changing impedance, due to breathing, affects the measured AC signal that is received (209) at the rest of patches 206.

Using the AC signals measured by meters 210, processor 56 of Fig. 1 applies an electrical model to compute the above-described matrix **σ,** which, in the case of Fig. 2, is a 6x6 matrix. An example of a total impedance value processor 56 derives from **σ**, which is the reciprocal of the trace of matrix **σ**, R(t)=1/Tr(**σ**), where t is time, is shown in Fig. 3.

### ELECTRICAL DETECTION OF END-OF-EXPIRIUM

Fig. 3 is a graph 300 of the total impedance R(t), 333, of an actual patient undergoing cardiac ablation, in accordance with an example of the present disclosure. Total impedance 333 is shown along a reference method graph (334) that electrically measures end-of-expirium, derived using the methods of U.S. Patent 10,524,692, which serves as a verification reference. As seen, there is good agreement between the graphs on the timings of minima 302 of impedance.

One possible reason to consider using the disclosed method (e.g., graph 333) in cardiac ablation is its simplicity compared with the method of U.S. Patent 10,524,692. Another possible reason to consider using the disclosed method in cardiac ablation is that graph 333 shows that the disclosed method is less affected by the high voltage or current signals that cardiac ablation applies during time windows (seen as "ones" in curve 335).

### METHOD OF DETECTING END-OF-EXPIRIUM USING BODY IMPEDANCES

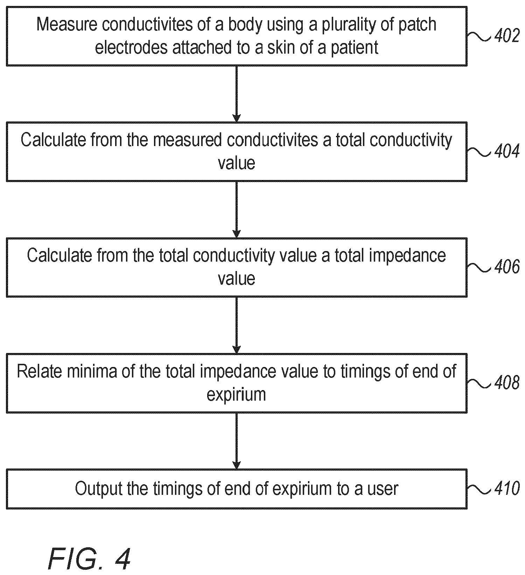

Fig. 4 is a flow chart that schematically illustrates a method and algorithm to electrically detect end-of-expirium, in accordance with an example of the present disclosure. The algorithm, according to the present embodiment, carries out a process that begins, with conductivities measurement step 402, by measuring conductivities of a body of a patient using the disclosed technique that involves patch electrodes, as shown implemented by way of example in Fig. 2.

Next, at a total conductivity calculation step 404, a processor uses the measured conductivities to calculate a total conductivity value, denoted as Tr(**σ**). Next, in a total impedance calculation step 406, the processor derives a measure of the total impedance, denoted as 1/Tr(**σ**). Steps 404 and 406 are brought, in some excess detail, for clarity of the exemplified method, though these steps may be reduced into a single step in other examples.

In minima relating step 408, the processor relates the minima of 1/Tr(**σ**) to timings of end-of-expirium, for example, by tagging the minima for use in cardiac ablation.

Finally, at an outputting step 410, the processor outputs the timings of end-of-expirium as an algorithm (e.g., a cardiac ablation algorithm). The processor outputs the timings of end-of-expirium by displaying curve 333 on a display device 27.

The example flow chart shown in Fig. 4 is chosen purely for the sake of conceptual clarity. The present example may also comprise additional steps of the algorithm, such as acquiring electrocardiograms, which have been omitted from the disclosure herein purposely to provide a more simplified flow chart.

Although the examples described herein mainly address cardiac diagnostic applications, the methods and systems described herein can also be used in other medical applications.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art. The scope of the invention is defined by the claims.

## Claims

1. A method, comprising:
receiving multiple AC signals from a plurality of patch electrodes (38) attached to a patient (23) as a function of time, wherein each electrode acts as a transmitter of an AC signal in a unique frequency while the other electrodes act as receivers, and wherein the multiple AC signals are received without using a probe within the body of the patient;
calculating, based on the received AC signals, a combined conductivity matrix, wherein the combined conductivity matrix describes the combined conductance of the electrodes and the patient's body's conductance;
calculating a total conductivity value as a trace of the combined conductivity matrix;
calculating a total impedance value from the total conductivity value, as a function of time, wherein the total impedance value is calculated as the reciprocal of the total conductivity value;
relating temporal minima of the total impedance value with timings of end-of-expirium of the patient; and
outputting the timings of end-of-expirium.

2. The method according to claim 1, wherein the plurality of electrodes comprise electrodes of an electrical position-tracking system.

3. The method according to claim 1 or claim 2, wherein outputting the timings comprises outputting the timings to a cardiac ablation algorithm.

4. The method according to any of claims 1 to 3, wherein outputting the timings comprises displaying the timings on a display device.

5. A system (10), comprising:
a plurality of patch electrodes (38, 206) configured to be attached to a body of a patient (23), wherein the system does not include a probe configured to be used within the body of the patient;
a plurality of AC signal sources (208) configured to apply an AC signal between each respective electrode and a common ground (214), wherein each of the AC signal sources transmit at a unique frequency;
a plurality of AC signal meters (210) configured to measure resulting AC signals between each of the electrodes and the common ground as a function of time; and
a processor (56), which is configured to:
receive the measured resulting AC signals;
calculate, based on the measured resulting AC signals, a combined conductivity matrix of the body, wherein the combined conductivity matrix describes the combined conductance of the electrodes and the patient's body's conductance,;
calculating a total conductivity value as the trace of the combined conductivity matrix;
calculate a total impedance value from total conductivity value, as a function of time, wherein the total impedance value is calculated as the reciprocal of the total conductivity;
relate temporal minima of the total impedance value with timings of end-of-expirium of the patient; and
output the timings of end-of-expirium to a user.

6. The system according to claim 5, wherein the plurality of electrodes comprise electrodes of an electrical position-tracking system.

7. The system according to claim 5 or claim 6, wherein the processor is further configured to apply a medical procedure to the patient at time-windows around the timings of end-of-expirium.

8. The system according to claim 7, wherein the medical procedure comprises cardiac ablation.

9. The system according to any of claims 5 to 8, wherein the processor is configured to output the timings by outputting the timings to a cardiac ablation algorithm.

10. The system according to any of claims 5 to 8, wherein the processor is configured to output the timings by displaying the timings on a display device.

## Patentansprüche

1. Verfahren, umfassend:
Empfangen mehrerer Wechselstromsignale von einer Vielzahl von Patch-Elektroden (38), die an einem Patienten (23) angebracht sind, in Abhängigkeit von Zeit, wobei jede Elektrode als ein Sender eines Wechselstromsignals in einer eindeutigen Frequenz fungiert, während die anderen Elektroden als Empfänger fungieren, und wobei die mehreren Wechselstromsignale empfangen werden, ohne eine Sonde innerhalb des Körpers des Patienten zu verwenden;
Berechnen, basierend auf den empfangenen Wechselstromsignalen, einer kombinierten Leitfähigkeitsmatrix, wobei die kombinierte Leitfähigkeitsmatrix den kombinierten Leitwert der Elektroden und den Leitwert eines Körpers des Patienten beschreibt;
Berechnen eines Gesamtleitfähigkeitsbetrags als eine Spur der kombinierten Leitfähigkeitsmatrix;
Berechnen eines Gesamtimpedanzbetrags aus dem Gesamtleitfähigkeitsbetrag in Abhängigkeit von Zeit, wobei der Gesamtimpedanzbetrag als der Kehrwert des Gesamtleitfähigkeitsbetrags berechnet wird;
Verknüpfen zeitlicher Minima des Gesamtimpedanzbetrags mit Zeitpunkten eines Exspiriumsendes des Patienten; und
Ausgeben der Zeitpunkte des Exspiriumsendes.

2. Verfahren nach Anspruch 1, wobei die Vielzahl von Elektroden Elektroden eines elektrischen Positionsverfolgungssystems umfassen.

3. Verfahren nach Anspruch 1 oder 2, wobei das Ausgeben der Zeitpunkte das Ausgeben der Zeitpunkte an einen Herzablationsalgorithmus umfasst.

4. Verfahren nach einem der Ansprüche 1 bis 3, wobei das Ausgeben der Zeitpunkte ein Anzeigen der Zeitpunkte auf einer Anzeigevorrichtung umfasst.

5. System (10), umfassend:
eine Vielzahl von Patch-Elektroden (38, 206), die konfiguriert sind, um an einem Körper eines Patienten (23) angebracht zu werden, wobei das System keine Sonde einschließt, die konfiguriert ist, um innerhalb des Körpers des Patienten verwendet zu werden;
eine Vielzahl von Wechselstromsignalquellen (208), die konfiguriert sind, um ein Wechselstromsignal zwischen jeder jeweiligen Elektrode und einer gemeinsamen Masse (214) anzuwenden, wobei jede der Wechselstromsignalquellen mit einer eindeutigen Frequenz sendet;
eine Vielzahl von Wechselstromsignalmessern (210), die konfiguriert sind, um resultierende Wechselstromsignale zwischen jeder der Elektroden und der gemeinsamen Masse in Abhängigkeit von Zeit zu messen; und
einen Prozessor (56), der konfiguriert ist zum:
Empfangen der gemessenen resultierenden Wechselstromsignale;
Berechnen, basierend auf den gemessenen resultierenden Wechselstromsignalen, einer kombinierten Leitfähigkeitsmatrix des Körpers, wobei die kombinierte Leitfähigkeitsmatrix den kombinierten Leitwert der Elektroden und den Leitwert des Körpers des Patienten beschreibt,;
Berechnen eines Gesamtleitfähigkeitsbetrags als die Spur der kombinierten Leitfähigkeitsmatrix;
Berechnen eines Gesamtimpedanzbetrags aus dem Gesamtleitfähigkeitsbetrag in Abhängigkeit von Zeit, wobei der Gesamtimpedanzbetrag als der Kehrwert der Gesamtleitfähigkeit berechnet wird;
Verknüpfen zeitlicher Minima des Gesamtimpedanzbetrags mit Zeitpunkten des Exspiriumsendes des Patienten; und
Ausgeben der Zeitpunkte des Exspiriumsendes an einen Benutzer.

6. System nach Anspruch 5, wobei die Vielzahl von Elektroden Elektroden eines elektrischen Positionsverfolgungssystems umfassen.

7. System nach Anspruch 5 oder 6, wobei der Prozessor ferner konfiguriert ist, um eine medizinische Prozedur auf den Patienten in Zeitfenstern um die Zeitpunkte des Exspiriumsendes herum anzuwenden.

8. System nach Anspruch 7, wobei die medizinische Prozedur eine Herzablation umfasst.

9. System nach einem der Ansprüche 5 bis 8, wobei der Prozessor konfiguriert ist, um die Zeitpunkte durch Ausgeben der Zeitpunkte an einen Herzablationsalgorithmus auszugeben.

10. System nach einem der Ansprüche 5 bis 8, wobei der Prozessor konfiguriert ist, um die Zeitpunkte durch Anzeigen der Zeitpunkte auf einer Anzeigevorrichtung auszugeben.

## Revendications

1. Procédé, comprenant :
la réception de multiples signaux CA à partir d'une pluralité d'électrodes patch (38) fixées à un patient (23) en fonction du temps, dans lequel chaque électrode agit comme un émetteur d'un signal CA à une fréquence unique tandis que les autres électrodes agissent comme des récepteurs, et dans lequel les multiples signaux CA sont reçus sans utiliser de sonde à l'intérieur du corps du patient ;
le calcul, sur la base des signaux CA reçus, d'une matrice de conductivité combinée, dans lequel la matrice de conductivité combinée décrit la conductance combinée des électrodes et la conductance du corps du patient ;
le calcul d'une valeur de conductivité totale en tant que trace de la matrice de conductivité combinée ;
le calcul d'une valeur d'impédance totale à partir de la valeur de conductivité totale, en fonction du temps, dans lequel cette valeur d'impédance totale est calculée comme l'inverse de la valeur de conductivité totale ;
la mise en relation de minima temporels de la valeur d'impédance totale avec des instants de fin d'expiration du patient ; et
la délivrance en sortie des instants de fin d'expiration.

2. Procédé selon la revendication 1, dans lequel la pluralité d'électrodes comprend des électrodes d'un système de suivi de position électrique.

3. Procédé selon la revendication 1 ou la revendication 2, dans lequel la délivrance en sortie des instants comprend la délivrance en sortie des instants vers un algorithme d'ablation cardiaque.

4. Procédé selon l'une quelconque des revendications 1 à 3, dans lequel la délivrance en sortie des instants comprend l'affichage des instants sur un dispositif d'affichage.

5. Système (10), comprenant :
une pluralité d'électrodes patch (38, 206) conçues pour être fixées au corps d'un patient (23), dans lequel le système ne comporte pas de sonde conçue pour être utilisée à l'intérieur du corps du patient ;
une pluralité de sources de signaux CA (208) configurées pour appliquer un signal CA entre chaque électrode respective et une masse commune (214), dans lequel chacune des sources de signal CA transmet à une fréquence unique ;
une pluralité de dispositifs de mesure de signal CA (210) configurés pour mesurer les signaux CA résultants entre chacune des électrodes et la masse commune en fonction du temps ; et
un processeur (56), qui est configuré pour :
recevoir les signaux CA résultants mesurés ;
calculer, sur la base des signaux CA résultants mesurés, une matrice de conductivité combinée du corps, dans lequel la matrice de conductivité combinée décrit la conductance combinée des électrodes et la conductance du corps du patient ;
calculer une valeur de conductivité totale en tant que trace de la matrice de conductivité combinée ;
calculer une valeur d'impédance totale à partir de la valeur de conductivité totale, en fonction du temps, dans lequel la valeur d'impédance totale est calculée comme l'inverse de la conductivité totale ;
mettre en relation des minima temporels de la valeur d'impédance totale avec des instants de fin d'expiration du patient ; et
délivrer en sortie les instants de fin d'expiration à un utilisateur.

6. Système selon la revendication 5, dans lequel la pluralité d'électrodes comprend des électrodes d'un système de suivi de position électrique.

7. Système selon la revendication 5 ou la revendication 6, dans lequel le processeur est en outre configuré pour appliquer une procédure médicale au patient à des fenêtres temporelles autour des instants de fin d'expiration.

8. Système selon la revendication 7, dans lequel la procédure médicale comprend une ablation cardiaque.

9. Système selon l'une quelconque des revendications 5 à 8, dans lequel le processeur est configuré pour délivrer en sortie les instants en délivrant en sortie les instants à un algorithme d'ablation cardiaque.

10. Système selon l'une quelconque des revendications 5 à 8, dans lequel le processeur est configuré pour délivrer en sortie les instants en affichant les instants sur un dispositif d'affichage.
